# EUROPEAN PATENT APPLICATION

(11) **EP 2 113 565 A1**
(43) Date of publication of application: **04.11.2009**
(21) Application number: 08711482.3
(22) Date of filing: 18.02.2008
(51) Int. Cl.: C12N 15/09, C12M 1/00, C12N 5/10

(54) **CONDUCTIVE SUBSTRATE FOR NUCLEIC ACID DELIVERY AND METHOD FOR DELIVERING NUCLEIC ACID**

(30) Priority: 19.02.2007 JP 2007038067
(71) Applicant: Kyoto University, Kyoto-shi Kyoto 606-8501 (JP)
(72) Inventor: IWATA, Hiroo, Kyoto-shi Kyoto 606-8507 (JP); INOUE, Yuuki, Kyoto-shi Kyoto 606-8507 (JP)
(74) Representative: Kalhammer, Georg
(86) International application number: PCT/JP2008/052658
(87) International publication number: WO 2008/102728

(57) **Abstract**

A conductive substrate for introducing a nucleic acid into a cell, which comprises a carbon nanotube with a carboxyl group, and a nucleic acid, the carbon nanotube and the nucleic acid being loaded on an electrode substrate with a cationic surface.

SEQUENCE LISTING

<110>  Kyoto University

<120>  Electric conductive substrate and method for introducing nucleic acid

<130>  K29F2680

<150>  JP2007-038067

<151>  2007-02-19

<160>  4

<170>  PatentIn version 3.1

<210>  1
<211>  22
<212>  RNA
<213>  Artificial

<220>
<223>  siRNA specifically suppressing the expression of GFP sequence

<400>  1
gcaagcugac ccugaaguuc au                                                22

<210>  2
<211>  22
<212>  RNA
<213>  Artificial

<220>
<223>  siRNA specifically suppressing the expression of GFP sequence

<400>  2
gaacuucagg gucagcuugc cg                                                22

<210>  3
<211>  19
<212>  RNA
<213>  Artificial

<220>
<223>   siRNA non-specific for GFP sequencee

<400>  3
uucuccgaac gugucacgu                                                    19

<210>  4
<211>  19
<212>  RNA
<213>  Artificial

<220>
<223>  siRNA non-specific for GFP sequence

<400>  4
acgugacacg uucggagaa                                                    19

## Description

### TECHNICAL FIELD

The present invention relates to a conductive substrate and a method for introducing nucleic acid, and more specifically, relates to a conductive substrate and a method for introducing a nucleic acid into cells by electroporation.

### BACKGROUND ART

Methods for introducing nucleic acids into cells are utilized for elucidation of functions of genes and proteins encoded by genes and for mass production of recombinant proteins. Further, such methods for introducing genes into cells are also utilized for introducing genes of objective enzymes or cytokines into cells of a patient and allowing the genes to produce objective substances in the body, thereby to provide treatment for diseases.

Among various methods for introducing nucleic acids into living cells, electroporation enables relatively efficient introduction of nucleic acids into cells and is applicable to a wide range of cells. However, general methods for gene transfection including the conventional electroporation are carried out in a liquid-phase homogeneous system, and due to operational limitation, the only option was to introduce the same set of genes into all the cells. The Human Genome Project has revealed base sequences of a wide variety of genes including those of which functions are unknown. Under the circumstances, analysis of the expression and the suppression of the functions thereof at the cellular level is needed. Accordingly, development of technology to efficiently introduce a wide variety of genes into different cells simultaneously is desired.

As a method for expeditiously introducing a wide variety of genes into different cells, Sabatini et al. have developed a transfection array wherein a wide variety of genes are arranged in parallel on a substrate and the genes are introduced all at once into separate cells adherent to the substrate (reverse transfection) (J. Ziauddin, D. M. Sabatini, Nature 411: 107-110; 2001). In the method employing this transfection array, adherent cells are cultured on a glass substrate onto which a wide variety of genes integrated into expression vectors are printed, and the genes are introduced into and expressed in the cells, enabling simultaneous analysis of the functions of various kinds of genes. However, since this method employs lipofection for transfection, problems attributable to lipofection cannot be eliminated. The timing of gene transfection cannot be controlled and the transfection efficiency is low.

The inventors developed a transfection array wherein nucleic acids are introduced into cells by electroporation (F. Yamauchi, K. Kato, H. Iwata, Nucleic Acids Res. 32: e187; 2004 and WO 2005/035755). This transfection array introduces nucleic acids into cells by loading nucleic acids onto an electrode surface, making cells adhere to the obtained nucleic acid-loaded electrode surface, and applying electrical pulses to the adherent cells. According to this method, genes can be efficiently introduced into cells without much cell damage, at desirable timing and at desirable sites. Furthermore, a wide variety of genes can be introduced into cells simultaneously. In recent years, however, in order to analyze the functions of a large number of genes efficiently, densification of such a transfection array is needed. As the transfection array is densified, each spot on the array becomes smaller in size, resulting in reduced number of cells which adhere to each spot. For this reason, more efficient transfection into cells is needed in order to conduct a significant functional analysis using only a small number of cells.

### DISCLOSURE OF THE INVENTION

### PROBLEMS TO BE SOLVED BY THE INVENTION

It is an object of the present invention to provide a conductive substrate and a method for introducing a nucleic acid into cells by electroporation. More specifically, it is an object to provide a conductive substrate and a method for introducing a nucleic acid into cells, wherein modification of the conductive substrate improves transfection efficiency in electroporation and thereby enables a significant functional analysis using only a small number of cells.

### MEANS FOR SOLVING THE PROBLEMS

The present inventors have made attempts to improve a method for introducing genes by a conventional electroporation, and as a result, have found that modifying an electrode surface for electroporation with carbon nanotubes (CNTs) markedly improves transfection efficiency into cells adherent to the electrode. That is, the present inventors have found that loading carbon nanotubes and nucleic acids onto an electrode surface, making cells adhere to the obtained nucleic acid-loaded electrode surface, and then applying electrical pulses to the adherent cells improve transfection efficiency because electric fields generated by the electrical pulses concentrate on the carbon nanotubes and thereby give strong local electric fields to the cells so that pores are efficiently made in cell membranes, and have completed the present invention. The completion of the present invention has enabled a significant functional analysis using only a small number of cells as well as preparation of transfection array with which functional analysis of a wide variety of nucleic acids can be conducted simultaneously.

That is, the present invention relates to:
(1) a conductive substrate for introducing a nucleic acid into a cell, which comprises a carbon nanotube with a carboxyl group, and a nucleic acid, the carbon nanotube and the nucleic acid being loaded on an electrode substrate with a cationic surface;
(2) the conductive substrate for introducing a nucleic acid according to the above (1), wherein the carbon nanotube with a carboxyl group, and the nucleic acid are loaded on the cationic surface of the electrode substrate directly or via a cationic polymer;
(3) the conductive substrate for introducing a nucleic acid according to the above (2), wherein the carbon nanotube with a carboxyl group is made to adsorb onto the cationic surface of the electrode substrate only once, or the carbon nanotube with a carboxyl group and the cationic polymer are alternately laminated on the cationic surface of the electrode substrate in the order of a carbon nanotube with a carboxyl group, a cationic polymer, and a carbon nanotube with a carboxyl group;
(4) the conductive substrate for introducing a nucleic acid according to the above (2) or (3), wherein the nucleic acid is made to adsorb only once, or the nucleic acid and the cationic polymer are alternately laminated in the order of a nucleic acid, a cationic polymer, and a nucleic acid;
(5) the conductive substrate for introducing a nucleic acid according to any one of the above (1) to (4), wherein the electrode substrate with a cationic surface has a substrate coated with a metal selected from gold, platinum and aluminum, or with a transparent semiconductor film of indium tin oxide (ITO);
(6) the conductive substrate for introducing a nucleic acid according to any one of the above (1) to (5), wherein the electrode substrate with a cationic surface has a substrate coated with a gold thin film;
(7) the conductive substrate for introducing a nucleic acid according to the above (6), wherein the electrode substrate coated with a gold thin film is a glass substrate or a polymer substrate on which gold is deposited;
(8) the conductive substrate for introducing a nucleic acid according to any one of the above (5) to (7), wherein the thickness of the metal or ITO on the surface of the electrode substrate is 1 nm to 200 µm;
(9) the conductive substrate for introducing a nucleic acid according to any one of the above (1) to (8), wherein the electrode substrate with a cationic surface has a monolayer film of a thiol compound, a disulfide compound, a sulfide compound or a silane coupling agent having a cationic functional group at the terminal, the monolayer film being formed on the surface of an electrode, or wherein the surface of the electrode substrate is modified with a cationic polymer;
(10) the conductive substrate for introducing a nucleic acid according to the above (9), wherein the surface of the electrode substrate with a cationic surface is modified with a cationic polymer has a monolayer film of a thiol compound, a disulfide compound, a sulfide compound or a silane coupling agent having an anionic functional group at the terminal, the monolayer film being formed on the surface of an electrode, and a cationic polymer is adsorbed onto the surface of the monolayer film;
(11) the conductive substrate for introducing a nucleic acid according to the above (9), wherein the surface of the electrode substrate with a cationic surface is modified with a cationic polymer has a monolayer film of a thiol compound, a disulfide compound, a sulfide compound or a silane coupling agent having a cationic functional group at the terminal, the monolayer film being formed on the surface of an electrode, and an anionic polymer is adsorbed onto the surface of the monolayer film, and further a cationic polymer is adsorbed thereonto;
(12) the conductive substrate for introducing a nucleic acid according to any one of the above (1) to (11), wherein the electrode substrate with a cationic surface has a micropatterned surface; and
(13) the conductive substrate for introducing a nucleic acid according to any one of the above (1) to (12), wherein the nucleic acid is at least one kind selected from the group consisting of DNAs, RNAs, antisense nucleic acids, siRNAs, and expression vectors therefor.

The present invention also relates to:
(14) a method for introducing a nucleic acid into a cell by electroporation, which comprises the steps of:
   (A) loading a carbon nanotube with a carboxyl group, and a nucleic acid onto the surface of an electrode;
   (B) making a cell adhere to the surface of the obtained nucleic acid-loaded electrode; and
   (C) applying an electrical pulse to the adherent cell;
(15) a method for introducing a nucleic acid into a cell by electroporation, which comprises the steps of:
   (a) providing an electrode substrate with a cationic surface;
   (b) loading a carbon nanotube with a carboxyl group, and a nucleic acid onto the surface of the electrode substrate with a cationic surface;
   (c) making a cell adhere to the surface of the nucleic acid-loaded electrode substrate obtained in step (b); and
   (d) applying an electrical pulse to the cell; (16) the method according to the above (15), wherein the step (b) is carried out by loading a carbon nanotube with a carboxyl group onto the surface of the electrode substrate with a cationic surface and subsequently loading a nucleic acid thereonto;
(17) the method according to the above (16), wherein the step (b) is carried out by making a carbon nanotube with a carboxyl group adsorb onto the cationic surface of an electrode substrate only once, or making carbon nanotube with a carboxyl group, and cationic polymer alternately adsorb onto the surface in the order of carbon nanotube with a carboxyl group, cationic polymer, and carbon nanotube with a carboxyl group, by an alternate adsorption method;
(18) the method according to the above (16) or (17), wherein the step (b) is carried out by making a nucleic acid adsorb only once, or making nucleic acid and cationic polymer alternately adsorb in the order of nucleic acid, cationic polymer, and nucleic acid, by an alternate adsorption method;
(19) the method according to any one of the above (15) to (18), wherein the electrode substrate with a cationic surface has a substrate coated with a metal selected from gold, platinum and aluminum, or with a transparent semiconductor film of indium tin oxide (ITO);
(20) the method according to any one of the above (15) to (19), wherein the electrode substrate with a cationic surface has a substrate coated with a gold thin film;
(21) the method according to the above (20), wherein the electrode substrate coated with a gold thin film is a glass substrate or a polymer substrate on which gold is deposited;
(22) the method according to any one of the above (15) to (21), wherein the electrode substrate with a cationic surface has a monolayer film of a thiol compound, a disulfide compound, a sulfide compound or a silane coupling agent having a cationic functional group at the terminal, the monolayer film being formed on the surface of an electrode, or wherein the surface of the electrode substrate is modified with a cationic polymer; (23) the method according to the above (22), wherein the surface of the electrode substrate with a cationic surface modified with a cationic polymer has a monolayer film of a thiol compound, a disulfide compound, a sulfide compound or a silane coupling agent having an anionic functional group at the terminal, the monolayer film being formed on the surface of an electrode, and a cationic polymer is adsorbed onto the surface of the monolayer film;
(24) the method according to the above (22), wherein the surface of the electrode substrate with a cationic surface modified with a cationic polymer has a monolayer film of a thiol compound, a disulfide compound, a sulfide compound or a silane coupling agent having a cationic functional group at the terminal, the monolayer film being formed on the surface of an electrode, and an anionic polymer is adsorbed onto the surface of the monolayer film, and further a cationic polymer is adsorbed thereonto; and
(25) the method according to any one of the above (15) to (24), wherein the electrode substrate with a cationic surface has a micropatterned surface.

### EFFECT OF THE INVENTION

According to the invention, provided is a conductive substrate and a method for introducing a nucleic acid by electroporation, wherein a significant functional analysis can be carried out using only a small amount of nucleic acid vector and a small number of cells because a nucleic acid such as a gene can be introduced into cells with high transfection efficiency without damaging the cells, a nucleic acid can be introduced at desirable timing and at desirable sites, and functional analysis of a wide variety of nucleic acids can be conducted simultaneously.

### BRIEF DESCRIPTION OF DRAWINGS

Fig. 1 shows an arrangement plan of the conductive substrate of the present invention for introducing a nucleic acid into adherent cells and a system for applying an electric pulse to the substrate.
Fig. 2 shows an IR-RAS chart of substrates produced at different CNT-COOH dispersion concentrations. (a) CNT-COOH dispersion concentration: 0.1 mg/mL, (b) CNT-COOH dispersion concentration: 0.01 mg/mL, (c) CNT-COOH dispersion concentration: 0.001 mg/mL
Fig. 3 shows phase contrast microscope images (upper left), fluorescence microscope images (upper right) and fluorescence intensity distributions (below), 48 hours after electroporation of cells adherent to each electrode. (a) CNT/PEI25000/COOH-SAM electrode, (b) PEI25000/COOH-SAM electrode, Scale bar: 500 µm
Fig. 4 shows phase contrast microscope images (upper) and fluorescence microscope images (lower), 48 hours after electroporation of cells adherent to each electrode. (a) CNT/PEI2000/Au electrode, (b) PEI2000/Au electrode, Scale bar: 500 µm
Fig. 5 shows phase contrast microscope images (upper) and fluorescence microscope images (lower), 48 hours after electroporation of cells adherent to each electrode. (a) CNT/PEI2000/ITO electrode, (b) PEI2000/ITO electrode, Scale bar: 500 µm
Fig. 6 shows a fluorescence microscope image, 48 hours after electroporation of cells adherent to a CNT-COOH-loaded electrode. Scale bar: 1 mm
Fig. 7 shows fluorescence microscope images of cells in which GFP expression was suppressed by siRNAs introduced thereinto. Scale bar: 1 mm
Fig. 8 shows phase contrast microscope images (upper left), fluorescence microscope images (upper right), and fluorescence intensity distributions (below) 48 hours after electroporation of cells adherent to each CNT-COOH/L-PEI25000 alternately laminated electrode. (a) CNT/L-PEI25000/COOH-SAM electrode, (b) L-PEI25000/(CNT/L-PEI25000)₁/COOH-SAM electrode, (c) (CNT/L-PEI25000)₅/COOH-SAM electrode, Scale bar: 500 µm

### DESCRIPTION OF SYMBOLS

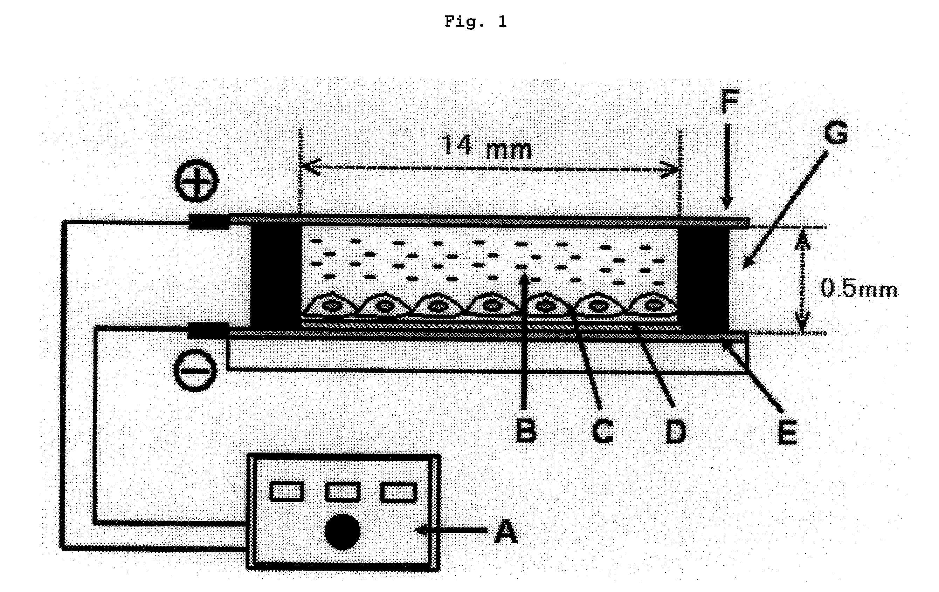

The meanings of the symbols in Fig. 1 are as follows.
- A: Power supply
- B: Electroporation buffer
- C: Cell
- D: Composite layer of CNT-COOH and nucleic acid
- E: Gold thin film electrode (cathode)
- F: Gold electrode (anode)
- G: Silicone spacer

### DESCRIPTION OF PREFERRED EMBODIMENTS

An aspect of the present invention is a conductive substrate for introducing a nucleic acid to cells, wherein a carbon nanotube with a carboxyl group (hereinafter may be referred to as CNT-COOH) and a nucleic acid are loaded on an electrode substrate with a cationic surface.

A preferred embodiment of the present invention includes a conductive substrate for introducing a nucleic acid, wherein a carbon nanotube with a carboxyl group, and a nucleic acid are directly loaded on a cationic surface of an electrode substrate, or loaded via a cationic polymer on an anionic surface of an electrode substrate. Alternatively, the carbon nanotube with a carboxyl group may be adsorbed and loaded alternately with a cationic polymer (will be described later) on the surface of an electrode substrate so as to form a multilayer such as (CNT-COOH)-cationic polymer-(CNT-COOH)-cationic polymer-(CNT-COOH). That is, the carbon nanotube with a carboxyl group may be made to adsorb onto the cationic surface of the electrode substrate only once, or the carbon nanotube with a carboxyl group, and the cationic polymer may be alternately laminated on the surface in the order of a carbon nanotube with a carboxyl group, a cationic polymer, and a carbon nanotube with a carboxyl group.

Further, on the above-mentioned CNT-COOH-loaded substrate surface, a nucleic acid may be adsorbed and loaded alternately with a cationic polymer so as to form a multilayer such as nucleic acid-cationic polymer-nucleic acid-cationic polymer-nucleic acid. That is, the nucleic acid may be made to adsorb only once, or the nucleic acid and the cationic polymer may be alternately laminated in the order of a nucleic acid, a cationic polymer, and a nucleic acid. By making a multilayer of the nucleic acid, the amount of loaded nucleic acid can be increased, and thereby the transfection efficiency for cells will improve. The outermost layer (surface) of the conductive substrate for introducing a nucleic acid is preferably a layer of nucleic acid.

### Carbon nanotube with a carboxyl group

The "carbon nanotube with a carboxyl group" used herein is prepared by carboxylation of a carbon nanotube. The carbon nanotube used as a raw material may be a single walled nanotube (SWNT) or a multi walled nanotube (MWNT). The diameter and the length of the carbon nanotube are not particularly limited, but usually, the average diameter is about 1 to 50 nm, preferably about 1.4 to 20 nm, and more preferably about 2 to 15 nm, and the average length is about 50 nm to 1 cm, preferably about 100 to 2000 nm, and more preferably about 200 to 500 nm. As such a carbon nanotube, commercially available products, for example, Multi Wall Nanotubes (made by SES Research) etc. can be used.

The method for preparing the carbon nanotube with a carboxyl group may be a publicly known method, for example, a method of mixing a carbon nanotube with a mixed acid of concentrated sulfuric acid and concentrated nitric acid and then irradiating the mixture with ultrasonic wave. In the mixed acid, the ratio of the concentrated nitric acid to 1 part by volume of the concentrated sulfuric acid is preferably about 0.2 to 1 part by volume, and more preferably about 0.3 to 0.5 part by volume. The ratio of the mixed acid to 1 mg of the carbon nanotube is preferably about 0.01 to 100 mL, more preferably about 0.1 to 10 mL, and further more preferably 0.5 to 5 mL. The ultrasonic treatment time may be suitably set, and for example, may be about 2 hours at room temperature. The carbon nanotube with a carboxyl group which can be used for the present invention and the preparing method thereof are described in J. Liu et al., SCIENCE 280: 1253-1256; 1998, for example.

### Nucleic acid

The "nucleic acid" used in the present invention may be a polynucleotide, an oligonucleotide, a DNA or an RNA. The DNA may be a plasmid DNA, a cDNA, a genomic DNA, or a synthetic DNA. The "nucleic acid" includes DNA derivatives and RNA derivatives. The derivative means a nucleic acid having a phosphorothioate bond or a nucleic acid in which the phosphoric acid moiety, sugar moiety or base moiety of the internucleotide is chemically modified for preventing digestion by an enzyme.

The "nucleic acid" includes a plasmid DNA which encodes a bioactive protein effective in treating or improving disease symptoms, a plasmid DNA which encodes an immune-stimulating protein effective in preventing, treating or improving disease symptoms, and a part of these DNAs.

The "nucleic acid" includes an antisense nucleic acid also. The "antisense nucleic acid" is a DNA molecule or an RNA molecule which is complementary to at least one region of a specific mRNA molecule. The antisense nucleic acid is introduced into a cell, and then hybridized with a corresponding mRNA, thereby to form a double-stranded molecule. Since the cell does not translate a double-stranded mRNA, the antisense nucleic acid interferes with translation of the mRNA. The "nucleic acid" further includes siRNA (small double-stranded RNA provoking RNA interference). Further, the "nucleic acid" includes expression vectors for DNA, RNA, the antisense nucleic acid and siRNA.

When the nucleic acid is a plasmid DNA encoding a protein, the plasmid need to be constructed so as to express genetic code in cells after introduction into cells, and therefore, the plasmid DNA contains elements required for expressing an objective gene, such as a promoter. Further, when it is required to confirm whether or not the objective gene has been introduced into the cell, the plasmid DNA may further include a reporter gene in addition to the objective gene. In the present invention, one or more kinds of nucleic acids may be used.

The size of the "nucleic acid" is not particularly limited, but is 10 bp to 200 kbp in general, preferably 15 bp to 100 kbp, and more preferably 20 bp to 80 kbp.

A preferable method for loading a carbon nanotube with a carboxyl group, and a nucleic acid onto the surface of the electrode substrate with a cationic surface is a method in which a carbon nanotube with a carboxyl group is loaded onto the surface of the electrode substrate with a cationic surface and a nucleic acid is subsequently loaded onto the outermost layer. Examples of such a method include:
(1) a method which comprises providing an electrode substrate with a cationic surface, making a carbon nanotube with a carboxyl group adsorb onto the surface of the electrode substrate, and subsequently making a nucleic acid adsorb thereonto;
(2) a method which comprises providing an electrode substrate with a cationic surface, making a carbon nanotube with a carboxyl group, and a cationic polymer alternately adsorb onto the surface of the electrode substrate so as to form a multilayer such as (CNT-COOH)-cationic polymer-(CNT-COOH)-cationic polymer-(CNT-COOH), and subsequently making a nucleic acid adsorb thereonto;
(3) a method which comprises providing an electrode substrate with a cationic surface, making a carbon nanotube with a carboxyl group adsorb onto the surface of the electrode substrate, and subsequently making a nucleic acid and a cationic polymer alternately adsorb thereonto so as to form a multilayer such as nucleic acid-cationic polymer-nucleic acid-cationic polymer-nucleic acid;
(4) a method which comprises providing an electrode substrate with a cationic surface, making a carbon nanotube with a carboxyl group, and a cationic polymer alternately adsorb onto the surface of the electrode substrate so as to form a multilayer such as (CNT-COOH)-cationic polymer-(CNT-COOH)-cationic polymer-(CNT-COOH), and subsequently making a nucleic acid and a cationic polymer alternately adsorb thereonto so as to form a multilayer such as nucleic acid-cationic polymer-nucleic acid-cationic polymer-nucleic acid.
   In the embodiments (2) and (4), the outermost layer of the multilayer comprising CNT-COOH and a cationic polymer may be CNT-COOH or cationic polymer.

### Electrode substrate with a cationic surface

The electrode substrate with a cationic surface of the present invention may be selected from those in which the surface of an electrode substrate has positive charge, and the preferable examples include (I) an electrode substrate with a cationic surface having a monolayer film of a thiol compound, a disulfide compound, a sulfide compound or a silane coupling agent having a cationic functional group at the terminal, the monolayer film being formed on the surface of the electrode, and (II) an electrode substrate with a cationic surface wherein the surface of the electrode substrate is modified with a cationic polymer.

Preferable examples of (II) an electrode substrate with a cationic surface wherein the surface of the electrode substrate is modified with a cationic polymer include (II-i) an electrode substrate with a cationic surface having a monolayer film formed thereon of a thiol compound, a disulfide compound, a sulfide compound or a silane coupling agent having an anionic functional group at the terminal, and a cationic polymer is adsorbed onto the surface of the monolayer film; and (II-ii) an electrode substrate with a cationic surface having a monolayer film formed thereon of a thiol compound, a disulfide compound, a sulfide compound or a silane coupling agent having a cationic functional group at the terminal, and an anionic polymer is adsorbed onto the surface of the monolayer film, and further a cationic polymer is adsorbed thereonto.

The electrode substrate with a cationic surface of the above (I) can be produced, for example, by forming a monolayer of a thiol compound, a disulfide compound, a sulfide compound or a silane coupling agent having a cationic functional group at the terminal on the surface of an electrode substrate.

The electrode substrate with a cationic surface of the above (II-i) can be produced, for example, by 1) forming a monolayer of a thiol compound, a disulfide compound, a sulfide compound or a silane coupling agent having an anionic functional group at the terminal on the surface of an electrode substrate and 2) making a cationic polymer adsorb onto the surface of the monolayer. After making the cationic polymer adsorb, further an anionic polymer and a cationic polymer may be made to alternately adsorb so as to form a multilayer such as cationic polymer-anionic polymer-cationic polymer-anionic polymer-cationic polymer.

The electrode substrate with a cationic surface of the above (II-i) can be produced, for example, by 1) forming a monolayer of a thiol compound, a disulfide compound, a sulfide compound or a silane coupling agent having a cationic functional group at the terminal on the surface of an electrode substrate, 2) making an anionic polymer adsorb onto the surface of the monolayer, and subsequently 3) making a cationic polymer adsorb onto the surface of the anionic polymer. In the present invention, 2) and 3) may be alternately repeated two or more times so as to form a multilayer.

The "substrate" of the electrode substrate is, for example, a plate, a chip (including a micro chip), or an array to support the electrode. The base material thereof is not particularly limited as long as it is an insulating material capable of supporting the electrode, and examples of the base material include inorganic insulating materials such as glass, mica, quartz, alumina, sapphire, forsterite, silicon carbide, silicon oxide and silicon nitride; and organic polymeric insulating materials such as polyethylene, polypropylene, polyisobutylene, poly(ethylene terephthalate), unsaturated polyester, fluorine containing resin, poly(vinyl chloride), poly(vinylidene chloride), poly(vinyl acetate), poly(vinyl acetal), acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, poly(phenylene oxide) and polysulfone. Among these, inorganic insulating materials such as glass, mica, quartz, alumina, sapphire, forsterite, silicon carbide, silicon oxide, and silicon nitride are particularly preferred.

The "electrode" of the electrode substrate is not particularly limited as long as it can serve as an electrode in electroporation, and the preferable examples include an electrode made of metal material such as gold, silver, platinum, aluminum and copper; and a transparent electrode made of indium-tin oxide (ITO). Preferably, the electrode substrate with a cationic surface has a substrate directly coated with a metal selected from the group consisting of gold, platinum and aluminum, or (with) a transparent semiconductor film of indium-tin oxide (ITO) on the above-mentioned substrate. More preferably, the electrode substrate has a gold thin film.

Preferable examples of the electrode substrate comprising the above-mentioned "substrate" and "electrode" include those in which a metal electrode selected from the group consisting of gold, platinum and aluminum or an ITO electrode is provided on one side of a glass substrate, a polymer substrate or a mica substrate. More preferable examples include those in which a gold electrode is provided on one side of a glass substrate, a mica substrate or a polymer substrate.

The thickness of the metal or ITO film on the surface of the electrode substrate is preferably 1 nm to 200 µm. When the thickness is less than 1 nm, the conductivity may be insufficient. On the other hand, when the thickness is more than 300 µm, observation of cells with a microscope may become difficult. More preferred thickness is 50 to 100 nm.

A publicly known method may be used to provide an electrode on one side of a substrate, and examples of the method for providing the above-mentioned metal onto an inorganic insulating substrate include a vacuum deposition method, a spattering method, an ion injection method, a plating method and the like. According to a preferable embodiment of the present invention, the electrode substrate coated with a gold thin film is a glass substrate or a polymer substrate onto which gold is deposited.

Examples of the thiol compound having an anionic functional group at the terminal which is used for forming a monolayer on the surface of an electrode include a thiol compound represented by the general formula (1) below:

R¹-(CH₂)ₙ-SH (1)

(wherein R¹ denotes an anionic functional group and n denotes an integer of 1 to 40). Preferable examples of the compound (1) include those in which an anionic functional group R¹ is a carboxyl group, a phosphate group, a sulfonic group, or a phosphonic acid group and n is 1 to 40, preferably 7 to 18, and more preferably 10 to 16.

Instead of the thiol compounds represented by the general formula (1), a disulfide compound or a sulfide compound represented by the following general formula (1A) or (1B):

R¹-(CH₂)ₙ-S-S-(CH₂)ₘ-R¹ (1A)

R¹-(CH₂)ₙ-S-(CH₂)ₘ-R¹ (1B)

may be used (wherein R¹ and n have the same meanings as defined above, and m denotes an integer of 1 to 40). Here, m is preferably 7 to 18, and more preferably 10 to 16. The disulfide compound or the sulfide compound may be symmetric or asymmetric, but a symmetric type, which can form a uniform monolayer, is preferred.

To form a monolayer film on the surface of the electrode substrate, a silane coupling agent having an anionic functional group at the terminal represented by the general formula (1C) below:

R¹-(CH₂)ₚ-Si(OR)₃ (1C)

(wherein R¹ has the same meaning as defined above, R denotes an alkyl group having 1 to 4 carbon atoms, and p denotes an integer of 1 to 40) may be used. Here, p is preferably 7 to 18, and more preferably 10 to 16.

Specific examples of the preferable compound (1) or the disulfide or sulfide compound thereof include, 11-mercaptoundecanoic acid, 8-mercaptooctanoic acid, 15-mercaptohexadecanoic acid, 10-carboxydecyl disulfide and the like. Examples of the method for introducing an anionic functional group into the surface of an electrode substrate by the silane coupling method include a method in which the surface is treated with 2-(carbomethyloxy)ethyltrichlorosilane, and then hydrolysis is carried out to introduce a carboxyl group into the surface of the electrode substrate.

Also, examples of the thiol compound having a cationic functional group at the terminal which is used for forming a monolayer on the surface of an electrode include a thiol compound represented by the general formula (2) below:

R²-(CH₂)ₙ-SH (2)

(wherein R² denotes a cationic functional group and n has the same meaning as defined above). Examples of the cationic functional group denoted by R² include primary, secondary, tertiary and quaternary amino groups, and n denotes an integer of 1 to 40, preferably 7 to 18, and more preferably 10 to 16.

Instead of the thiol compounds represented by the general formula (2), a disulfide compound or a sulfide compound represented by the following general formula (2A) or (2B):

R²-(CH₂)ₙ-S-S-(CH₂)ₘ-R² (2A)

R²-(CH₂)ₙ-S-(CH₂)ₘ-R² (2B)

may be used (wherein R² and n have the same meanings as defined above, and m denotes an integer of 1 to 40). Here, m is preferably 7 to 18, and more preferably 10 to 16. The disulfide compound or the sulfide compound may be symmetric or asymmetric, but a symmetric type, which can form a uniform monolayer, is preferred.

To form a monolayer film on the surface of the electrode substrate, a silane coupling agent having a cationic functional group at the terminal represented by the general formula (2C or 3C) below:

R²-(CH₂)ₚ-Si(OR)₃ (2C)

R²-(CH₂)_{q}-NH-(CH₂)ᵣ-Si(OR)₃ (3C)

may be used (wherein R, R² and p have the same meanings as defined above, and q and r each denote an integer of 1 to 40). Here, q and r are preferably 7 to 18, and more preferably 10 to 16.

Specific examples of the preferable compound (2) or the disulfide or sulfide compound thereof include 11-amino-1-undecanethiol and the like. Also, specific examples of the silane coupling agent having a cationic functional group at the terminal represented by the general formula (2C) or (3C) include 3-aminopropyltrimethoxysilane, 3-aminopropyltriethoxysilane, N-(2-aminoethyl)-3-aminopropyltrimethoxysilane, N-(2-aminoethyl)-3-aminopropylmethyldimethoxysilane and the like.

To form a monolayer film on the surface of the electrode substrate, a conventional method may be used. For example, an electrode substrate may be immersed in a solution of the thiol compound (represented by the general formula (1) or (2)), the disulfide compound thereof (represented by the general formula (1A) or (2A)), the sulfide compound thereof (represented by the general formula (1B) or (2B)), or the silane coupling agent (represented by the general formula (1C), (2C) or (3C)). In this way, a monolayer with high density and high orientation is formed on the electrode.

When the thiol compound having an anionic functional group at the terminal (compound represented by the general formula (1)) or the disulfide or sulfide compound thereof (compound represented by the general formula (1A) or (1B)), or the silane coupling agent having an anionic functional group at the terminal (compound represented by the general formula (1C)) is used as the thiol compound, the electrode substrate with a cationic surface of the above (II-i) is obtained by making a cationic polymer adsorb onto the surface of the monolayer of these compounds.

The "cationic polymer" used herein may be any cationic polymer capable of adsorbing by electrostatic interactions (for example, ionic bond) onto the surface of a monolayer formed on the surface of an electrode. Examples of such cationic polymers include a polyethyleneimine, polyallylamine, polyvinylamine, polyvinylpyridine, aminoacetalized poly(vinyl alcohol), acrylic or methacrylic polymer having primary to quaternary amine at the terminal of the side chain (for example, poly(N,N-dimethylaminoethyl methacrylate) and the like), acid treated gelatin, polylysine, polyornithine, polyarginine, protamine, chitosan, DEAE-cellulose, DEAE-dextran and polyamidoamine dendrimer (cationic dendrimer) and the like. Among these, a polyethyleneimine and polyallylamine are particularly preferable.

The average molecular weight of the cationic polymer is usually 200 to 5,000,000, preferably 500 to 5,000,000, more preferably 600 to 100,000, and still more preferably 800 to 50,000. For example, in the case of polyethyleneimine, the average molecular weight is preferably 200 to 250,000, more preferably 600 to 100,000, and still more preferably 800 to 30,000. Further, in the case of polyallylamine, the average molecular weight is preferably 500 to 150,000, more preferably 600 to 100,000, and still more preferably 800 to 50,000.

The adsorption of the cationic polymer onto the surface of a monolayer can be carried out by bringing a solution of the cationic polymer into contact with the surface of the monolayer. For example, it can be preferably carried out by applying a solution of the cationic polymer dissolved in an appropriate buffer (for example, phosphate buffered saline) onto the surface of the monolayer and leaving it at room temperature. The concentration of the cationic polymer is not particularly limited, but preferably 0.1% to 10%. Thus, the monolayer and the cationic polymer are adsorbed by ionic interactions, and the electrode substrate with a cationic surface of the above (II-i) is provided.

Meanwhile, when the thiol compound having a cationic functional group at the terminal (compound represented by the general formula (2)) or the disulfide or sulfide compound thereof (compound represented by the general formula (2A) or (2B)), or the silane coupling agent having a cationic functional group at the terminal (compound represented by the general formula (2C) or (3C)) is used as the thiol compound, a carboxylated carbon nanotube is directly adsorbed onto the surface of the monolayer of these compounds so as to obtain a cationic substrate, or an anionic polymer is firstly adsorbed thereonto.

The "anionic polymer" used herein may be any anionic polymer capable of adsorbing by electrostatic interactions (for example, ionic bond) onto the surface of a monolayer formed on the surface of an electrode. Examples of such anionic polymers include synthetic polymers such as poly(acrylic acid), poly(methacrylic acid), poly(styrene sulfonic acid) and poly(2-acrylamido-2-methylpropanesulfonic acid), and natural polymers such as alginic acid, hyaluronic acid, chondroitin sulfate and alkali-treated gelatin.

The adsorption of the anionic polymer onto the surface of a monolayer can be carried out by bringing a solution of the anionic polymer into contact with the surface of the monolayer. For example, it can be preferably carried out by applying a solution of the anionic polymer dissolved in an appropriate buffer (for example, phosphate buffered saline) onto the surface of the monolayer and leaving it at room temperature. The concentration of the anionic polymer is not particularly limited, but preferably 0.1% to 10%.

Then, the electrode substrate with a cationic surface of the above (II-i) is obtained by making a cationic polymer adsorb onto the surface of the adsorbed anionic polymer. The cationic polymer used herein may be the same as those mentioned above, and adsorption can be carried out in the same manner as mentioned above.

Loading of a carbon nanotube with a carboxyl group, and a nucleic acid onto the surface of the electrode substrate with a cationic surface
The conductive substrate of the present invention for introducing a nucleic acid can be obtained by loading a carbon nanotube with a carboxyl group, and a nucleic acid onto the surface of the electrode substrate with a cationic surface provided above.

The adsorption and loading of CNT-COOH onto the surface of an electrode substrate with a cationic surface can be carried out by bringing a dispersed solution of CNT-COOH in purified water into contact with the surface of the electrode substrate with a cationic surface. For example, it can be preferably carried out by immersing the electrode substrate with a cationic surface into a dispersed solution of CNT-COOH in purified water at room temperature. It is preferred that the electrode substrate with a cationic surface is stood in the dispersed solution of CNT-COOH in purified water during immersion because, in this case, deposition by gravity can be avoided and only electrostatic adsorption is involved in loading CNT-COOH. Also, it is preferred that the dispersed solution of CNT-COOH in purified water is stirred during immersion of the electrode substrate with a cationic surface. CNT-COOH not adsorbing onto the electrode substrate with a cationic surface can be removed by washing the surface of the electrode substrate with purified water. Thus, a CNT-COOH-loaded electrode substrate is obtained. The dispersed solution of CNT-COOH in purified water can be obtained by mixing CNT-COOH and purified water and irradiating the mixture with ultrasonic wave to disperse CNT-COOH in the purified water. The concentration of CNT-COOH in the purified water is preferably 0.01 to 500 mg/mL. When the concentration is less than 0.01 mg/mL, it is not possible to load a sufficient amount of CNT-COOH, and therefore the effect of the present invention cannot be obtained in some cases. When the concentration is more than 500 mg/mL, it becomes difficult to disperse CNT-COOH in purified water. The CNT-COOH concentration is more preferably 0.01 to 3.0 mg/mL, and is still more preferably 0.05 to 1.0 mg/mL.

The loading of a carbon nanotube with a carboxyl group onto the surface of an electrode substrate with a cationic surface can be confirmed by infrared reflection absorption spectroscopy (IR-RAS) of the substrate. That is, IR-RAS measurement of a CNT-COOH-loaded electrode substrate shows a carboxyl group absorption peak resulting from the loaded CNT-COOH.

It is not necessary that the entire surface of the obtained CNT-COOH-loaded electrode substrate is covered with the carbon nanotube. The surface of the substrate has positively charged portions. To the positively charged portions, nucleic acids, which will be described later, is adsorbed and loaded.

On the surface of the electrode substrate with a cationic surface, CNT-COOH and cationic polymer may be laminated in the order of CNT-COOH, cationic polymer, and CNT-COOH by an alternate adsorption method. As the cationic polymers, those mentioned above are preferred. The alternate adsorption of CNT-COOH and of cationic polymer here can be carried out in the same manner as mentioned above.

The adsorption of a nucleic acid onto the surface of the CNT-COOH-loaded electrode substrate with a cationic surface can be carried out by bringing a solution of the nucleic acid into contact with the surface of the electrode substrate with a cationic surface. For example, it can be preferably carried out by applying a solution of the nucleic acid dissolved in an appropriate buffer (for example, phosphate buffered saline) onto the surface of electrode substrate with a cationic surface and leaving it at room temperature. The nucleic acid not adsorbing onto the electrode substrate with a cationic surface can be removed by washing the surface of the electrode substrate with an appropriate buffer (for example, phosphate buffered saline). Thus, a nucleic acid-loaded electrode substrate (conductive substrate for introducing a nucleic acid) is obtained.

The nucleic acid-loaded electrode substrate (conductive substrate for introducing a nucleic acid) is obtained also by laminating nucleic acid and cationic polymer on the surface of an electrode substrate with a cationic surface in the order of nucleic acid, cationic polymer, and nucleic acid by an alternate adsorption method. The alternate adsorption of nucleic acid and of cationic polymer here can be carried out in the same manner as mentioned above.

In the present invention, as the conductive substrate for introducing a nucleic acid, a substrate having a micropatterned surface may be used. The micropatterned nucleic acid-loaded electrode substrate (conductive substrate for introducing a nucleic acid) can be prepared by partitioning the surface of an electrode substrate with a cationic surface using an appropriate method before loading of a carbon nanotube with a carboxyl group and a nucleic acid, and then loading multiple kinds of nucleic acids separately into each partition. When nucleic acids are introduced into cells by electroporation using such a micropatterned nucleic acid-loaded metal substrate (conductive substrate for introducing a nucleic acid), the sites where nucleic acids are introduced can be restricted.

Another aspect of the present invention is a method for introducing a nucleic acid into a cell by electroporation, which comprises the steps of:
(A) loading a carbon nanotube with a carboxyl group, and a nucleic acid onto the surface of an electrode;
(B) making a cell adhere to the surface of the obtained nucleic acid-loaded electrode; and
(C) applying an electrical pulse to the adherent cell.

Hereafter, each step will be described one by one.

### Step (A):

Examples of the method for loading a nucleic acid onto an electrode include a method, which comprises the steps of:
(a) providing an electrode substrate with a cationic surface; and
(b) loading a carbon nanotube with a carboxyl group, and a nucleic acid onto the surface of the electrode substrate with a cationic surface. Preferred embodiments of the electrode substrate with a cationic surface and the production method thereof are the same as those for the above-mentioned conductive substrate for introducing a nucleic acid. Also, the method for loading a carbon nanotube with a carboxyl group, and a nucleic acid onto the surface of the electrode substrate with a cationic surface and preferred embodiments thereof are the same as the above-mentioned method for producing a conductive substrate for introducing a nucleic acid and preferred embodiments thereof.

### Step (B):

This step is a step of making a cell adhere to the surface of the nucleic acid-loaded electrode substrate (conductive substrate for introducing a nucleic acid) obtained above.
The "cell" used in the present invention may be of any living thing (for example, any monocellular organism (for example, bacteria or yeast), multicellular organism (for example, an animal (for example, a vertebrate or an invertebrate), or plant (for example, a monocotyledonous plant, a dicotyledonous plant))) origin as long as it is an adhesive cell. For example, cells of vertebrate (for example, myxiniforme, petromyzoniforme, cartilaginous fish, teleost, amphibian, reptile, bird, mammal) origin are used, and cells of mammal (for example, monotreme, marsupialia, edentate, dermatopteran, chiropteran, carnivora, insectivora, proboscidean, perissodactyl, artiodactyl, tubulidentata, squamata, sirenian, cetacean, primate) origin are more preferably used. According to one embodiment, cells of primate (for example, chimpanzee, Japanese macaque, human) origin, especially cells of human origin are used. Specific examples of cells of human origin include human cell lines, such as human uterine cervix cancer cells (HeLa); human origin primary culture cells, such as human embryo kidney cells (HEK), human umbilical vein endothelial cells, human vascular endothelial cells, human aorta smooth muscle cells, human hepatocytes, human fibroblasts, human corneal epithelial cells, and human endothelium camerae anterioris cell; and human stem cells, such as mesenchymal stem cells, embryonic stem cells, and neural stem cells.

The method of making cells adhere onto the surface of a nucleic acid-loaded electrode substrate is not particularly limited, and for example, it can be carried out by applying a cell suspension onto the substrate, or by suspending cells in an appropriate culture medium, applying dropwise the obtained cell suspension onto the substrate and then incubating it under conditions optimized for the cells. However, it is preferable to carry out the cell cultivation on the nucleic acid-loaded electrode substrate from the viewpoints of transfection efficiency and restriction of the sites in a cell population into which nucleic acids are introduced. The cell cultivation allows the cells to proliferate and to adhere onto the surface of the substrate. Nonadhesive cells are preferably removed before applying electric pulse. Removal of nonadhesive cells can be carried out, for example, by exchanging a culture medium with a suitable buffer solution.

### Step (C):

The application of electric pulse to the cells can be carried out as follows. An electrode of a nucleic acid-loaded electrode substrate onto which cells adhere (referred to as a first electrode) and a counter electrode (referred to as a second electrode) facing the first electrode are provided. That is, cells are between the two electrodes. Electric pulse is generated between the first and second electrodes using an appropriate power supply. The electric pulse may be generated by alternate current or direct current, and preferably generated by direct current in general.

In the case of direct current, when the first electrode with loaded nucleic acids is used as a cathode (-), and the second electrode is used as an anode (+), and vice versa. Preferably, the first electrode is a cathode (-) and the second electrode is an anode (+). The material of the second electrode is not particularly limited so far as it is electroconductive. Examples of the material include metals such as gold, platinum, silver, copper, aluminum, stainless steel, tungsten, titanium, cobalt-chromium alloy, cobalt-chromium-molybdenum alloy (vitallium); carbon; and a transparent electrode material, indium-tin oxide (ITO).

The condition of electric pulsing, such as field strength, duration of an electric pulse, number of pulse application times and the like may be suitably selected depending on the kind of cells, nucleic acids to be introduced, cationic polymers on the substrate and the like. Usually, the field strength is 10 to 500 V/cm, preferably 25 to 400 V/cm, and more preferably 30 to 300 V/cm, the duration of an electric pulse is 1 to 99 ms, preferably 1 to 50 ms, and more preferably 3 to 20 ms, and the number of pulse application times is 1 to 99, preferably 1 to 5, and more preferably 1 to 3, but they are not limited thereto.

The timing of applying electric pulse is not particularly limited and can be suitably selected. That is, it is not necessary that electric pulses are applied immediately after cells are seeded on the nucleic acid-loaded substrate, incubated and made to adhere to the substrate. The pulses may be applied at any preferable time after cell adhesion, for example, anytime within about 4 to 5 days after cell seeding. This electric pulse application allows nucleic acids to be efficiently introduced into cells.

Fig. 1 exemplifies an arrangement plan of a nucleic acid-loaded electrode substrate (conductive substrate for introducing a nucleic acid) and a system for applying an electric pulse both of which are used to perform the method of the present invention. The details of the present invention are illustrated using the drawing. For example, a gold thin film electrode (E) is formed on the surface of a glass substrate. A composite layer (D) where CNT-COOH and nucleic acid are loaded is formed on the electrode, and cells (C) are made to adhere to the surface of the composite layer. Further, the cells are partitioned, for example, by a silicone spacer (G), and the well is filled with an electroporation buffer (B). A gold electrode (F) which is a counter electrode is provided on the silicon spacer (G) in parallel to the gold thin film electrode (E). The gold thin film electrode (E) and the gold electrode (F) are connected to a power supply (A) which generates electric pulses. In the example, the distance between the gold thin film electrode (E) and the gold electrode (F) is set to 0.5 mm and the well area is set to 14 × 14 mm². The nucleic acid (DNA) is introduced into the cells (C) by electric pulses generated by the power supply (A) by the use of the gold thin film electrode (E) as a cathode (-) and the gold electrode (F) as an anode (+).

As another aspect of the present invention, the above-mentioned nucleic acid introduction method can be carried out by the use of a substrate having a micropatterned surface. The micropatterned nucleic acid-loaded electrode substrate can be prepared by partitioning the surface of an electrode substrate with a cationic surface using an appropriate method before loading of a nucleic acid, and then loading multiple kinds of nucleic acids separately into each partition. When the present invention is implemented using such a micropatterned nucleic acid-loaded metal substrate, the sites where nucleic acids are introduced can be restricted.

### EXAMPLES

Hereafter, although examples explain the present invention further, the technical scope of the present invention is not limited by these examples.

### Example 1

### 1. Carboxylation of carbon nanotube (CNT)

A 40-mg multi-walled carbon nanotube (MWNT) (average diameter: 1.2 to 20 nm, average length: 100 to 2000 nm, number-of-layers: 5 to 20, SES Research, Texas, USA) was mixed with 40 mL of a mixed acid (concentrated sulfuric acid: concentrated nitric acid = 3:1 (volume ratio)). The mixture was irradiated with ultrasonic wave for 2 hours, neutralized with sodium hydroxide and filtered. The obtained residue was washed with purified water. The washed residue was freeze-dried to give carboxylated CNT (CNT-COOH).

### 2. Preparation of CNT-loaded electrode using gold surface on which an anionic self-assembled monolayer (SAM) film is formed

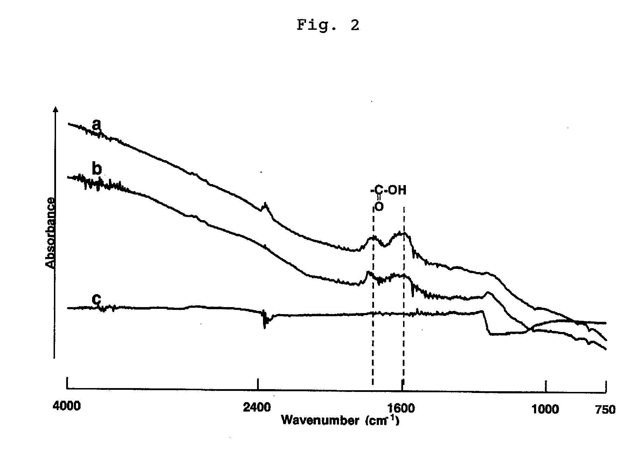

Onto a glass substrate (22 mm × 26 mm × 0.5 mm) washed with the piranha solution (concentrated sulfuric acid: hydrogen peroxide = 7:3 (volume ratio)), chromium and gold (Cr:Au = 1:49 (nm)) were thermally deposited. The substrate was immersed in a 1 mM solution of 11-mercaptoundecanoic acid in ethanol for 24 hours, and a film of self-assembled monolayer (SAM) with a carboxyl group (COOH-SAM) was obtained. The substrate was immersed in a 10 mg/mL solution of polyethyleneimine having a molecular weight of 25,000 (PEI25000) in physiological saline containing phosphoric acid (PBS solution, pH: 7.4) for 30 minutes, and a cationic substrate (PEI25000/COOH-SAM) was obtained. The substrate was immersed in a 0.001 mg/mL, 0.01 mg/mL, or 0.1 mg/mL dispersed solution of CNT-COOH in purified water for 2 hours, and CNT-COOH was electrostatically loaded on the substrate (CNT/PEI25000/COOH-SAM). Fig. 2 shows an IR-RAS chart of a CNT-COOH-loaded substrate thus obtained. As shown in Fig. 2, when the CNT-COOH concentration of the dispersed solution was 0.01 mg/mL or 0.1 mg/mL, absorption peaks resulting from the loaded CNT-COOH were observed, indicating that CNT was successfully loaded.

### 3. Gene introduction with CNT/PEI25000/COOH-SAM electrode

The obtained CNT/PEI25000/COOH-SAM electrode was sterilized with 70% ethanol and then dried in a clean bench. A silicone sheet (internal area: 14 mm × 14 mm, height: 0.5 mm) was pressure-bonded onto the electrode, and brought into contact with a 50 µg/mL solution of pEGFP-C1 (plasmid DNA containing a gene construct capable of expressing green fluorescent protein (GFP) under cytomegalovirus promoter control) in PBS (pH 7.4) for 30 minutes. The substrate was washed with PBS twice, and human embryo kidney (HEK293) cells were seeded thereon at 4.5 × 10⁴ cells/cm². The culture medium used was a minimum essential medium (MEM) supplemented with 0.1 mg/mL nonessential amino acids (alanine, asparagine, aspartic acid, glutamic acid, glycine, proline and serine), 10% fetal bovine serum (FBS), 100 U/mL penicillin and 0.1 mg/mL streptomycin. For sufficient cell adhesion, incubation was carried out in 5% CO₂ at 37°C for 24 hours. A PEI25000/COOH-SAM electrode was used for comparison.

The culture medium was replaced by PBS kept at 4°C and the counter electrode (a glass substrate onto which chromium and gold were thermally deposited at a film thickness of Cr:Au = 1:199 (nm)) was placed thereon. The electrode onto which cells adhere was connected to the negative pole, the counter electrode was connected to the positive pole, and pulse voltage was applied. The pulse voltage was applied at 13V for 10 ms once. After the pulse application, cells adherent onto the electrode were cultured in the culture medium for 48 hours.

### 4. Green fluorescent protein (GFP) expression analysis

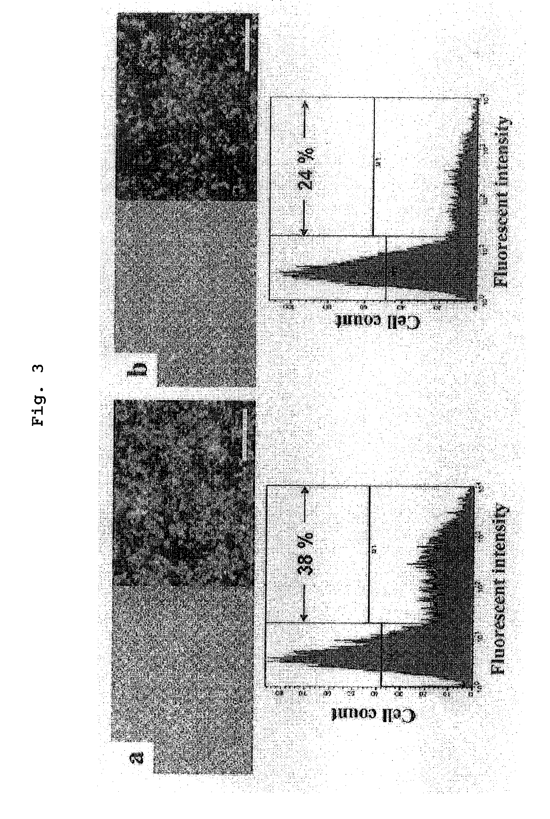

The GFP expression 48 hours after electroporation was observed with a fluorescence microscope, and the expression efficiency of GFP was computed by flow cytometry as more quantitative analysis. Fig. 3 shows the results (phase contrast microscope images: upper left, fluorescence microscope images: upper right, fluorescence intensity distributions (histograms obtained by flow cytometry): below, a: CNT/PEI25000/COOH-SAM electrode, b: PEI25000/COOH-SAM electrode). As shown in Fig. 3, the cells on the CNT/PEI25000/COOH-SAM electrode expressed more GFP as compared with the cells on the PEI25000/COOH-SAM electrode. The GFP expression efficiency using the CNT/PEI25000/COOH-SAM electrode and the PEI25000/COOH-SAM electrode were about 40% and about 20%, respectively. Loading CNT on the electrode significantly increased transfection efficiency.

### Example 2

### 1. Preparation of CNT-loaded electrode using gold surface

After preparing a substrate onto which a gold thin film was deposited according to 2 of Example 1, the substrate was immersed in a 10 mg/mL solution of polyethyleneimine having a molecular weight of 2,000 (PEI2000) in PBS (pH: 7.4) for 30 minutes, and a cationic substrate (PEI2000/Au) was obtained. The substrate was immersed in a 0.1 mg/mL dispersed solution of CNT-COOH in purified water for 2 hours, and CNT-COOH was loaded on the substrate (CNT/PEI2000/Au).

### 2. Gene introduction and gene expression analysis

According to 3 of Example 1, plasmid adsorption and cell seeding were performed using the obtained CNT/PEI2000/Au and PEI2000/Au electrodes. However, the concentration of the pEGFP-C1 solution and the contact time with the electrodes were 5 µg/mL and 3 hours, respectively.
According to 3 of Example 1, electroporation was performed using the above-mentioned electrodes. According to 4 of Example 1, the GFP expression was analyzed with a fluorescence microscope and by flow cytometry.

### 3. GFP expression analysis

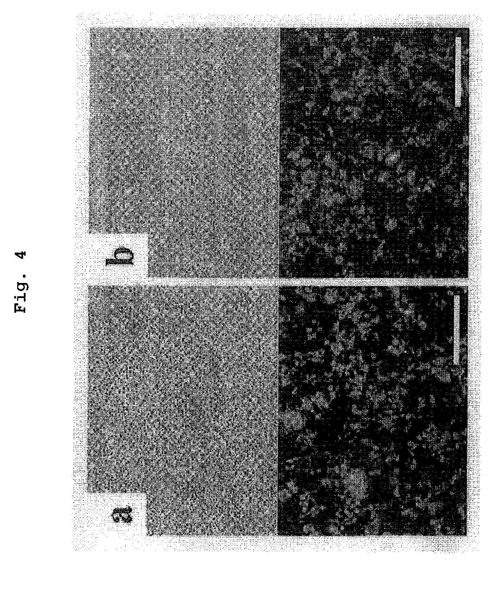

The GFP expression 48 hours after electroporation was observed with a fluorescence microscope. The results are shown in Fig. 4 (phase contrast microscope images: upper, fluorescence microscope images: below, a: CNT/PEI2000/Au electrode, b: PEI2000/Au electrode). As shown in Fig. 4, the cells on the CNT/PEI2000/Au electrode expressed more GFP as compared with the cells on the PEI2000/Au electrode. The GFP expression efficiency using the CNT/PEI2000/Au electrode and the PEI2000/Au electrode were about 40% and about 20%, respectively. Loading CNT on the electrode significantly increased transfection efficiency.

### Example 3

### 1. Preparation of CNT-loaded electrode using indium-tin oxide (ITO) surface

A glass substrate onto which an electrode material ITO was deposited was immersed in a 10 mg/mL solution of PEI2000 in PBS (pH: 7.4) for 30 minutes, and a cationic substrate (PEI2000/ITO) was obtained. The substrate was immersed in a 0.1 mg/mL dispersed solution of CNT-COOH in purified water for 2 hours, and CNT-COOH was loaded on the substrate (CNT/PEI2000/ITO).

### 2. Gene introduction and gene expression analysis

According to 3 of Example 1, plasmid adsorption and cell seeding were performed using the obtained CNT/PEI2000/ITO and PEI2000/ITO electrodes. However, the concentration of the pEGFP-C1 solution and the contact time with the electrodes were 5 µg/mL and 3 hours, respectively.

According to 3 of Example 1, electroporation was performed using the above-mentioned electrodes. However, the pulse voltage was applied at 33V for 10 ms once. According to 4 of Example 1, the GFP expression was analyzed with a fluorescence microscope and by flow cytometry.

### 3. GFP expression analysis

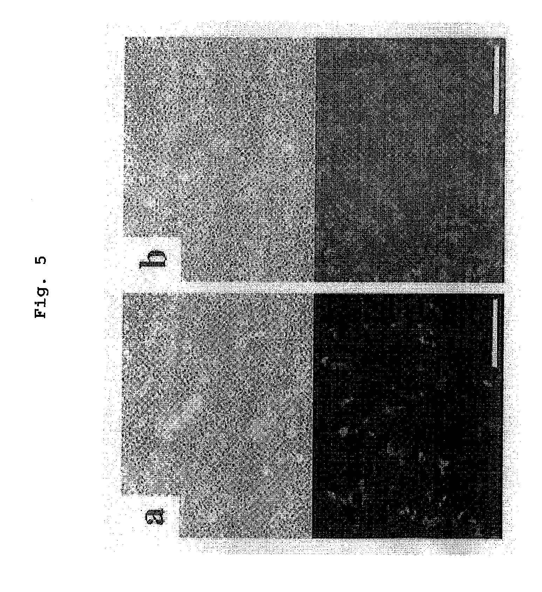

The GFP expression 48 hours after electroporation was observed with a fluorescence microscope. The results are shown in Fig. 5 (phase contrast microscope images: upper, fluorescence microscope images: below, a: CNT/PEI2000/ITO electrode, b: PEI2000/ITO electrode). As shown in Fig. 5, the cells on the CNT/PEI2000/ITO electrode expressed more GFP as compared with the cells on the PEI2000/ITO electrode. The GFP expression efficiency using the CNT/PEI2000/ITO electrode and the PEI2000/ITO electrode were about 45% and about 20%, respectively. Loading CNT on the electrode significantly increased transfection efficiency.

### Example 4

### 1. Preparation of CNT-loaded transfection array

A glass substrate onto which a gold thin film (Cr:Au = 1:49 (nm)) was thermally deposited was immersed in a 1mM solution of 1-hexadecanethiol in ethanol for 24 hours, and a SAM film with a methyl group (CH₃-SAM) was formed. UV irradiation was performed for 2 hours through a photomask having a transparent circular area having 25 circular spots 1 mm in diameter. As a result, a portion of the CH₃-SAM film was photodegraded, and gold was exposed. The substrate was immersed in a 1mM solution of 11-mercaptoundecanoic acid in ethanol for 1 hour, and COOH-SAM was formed on the exposed portion. The substrate was immersed in a 10 mg/mL solution of PEI2000 in PBS (pH: 7.4) for 30 minutes. The substrate was immersed in a 0.1 mg/mL dispersed solution of CNT-COOH for 2 hours, and CNT-loaded transfection array was prepared.

### 2. Site-specific transfection using CNT-loaded transfection array

In order to make pEGFP-C1 adsorb in an array form, a 5 µg/mL solution of pEGFP-C1 in PBS (pH 7.4) was spotted on the COOH-SAM portion. According to 3 of Example 1, pEGFP-C1 was introduced into HEK293 cells adherent onto the electrodes.

### 3. GFP expression analysis

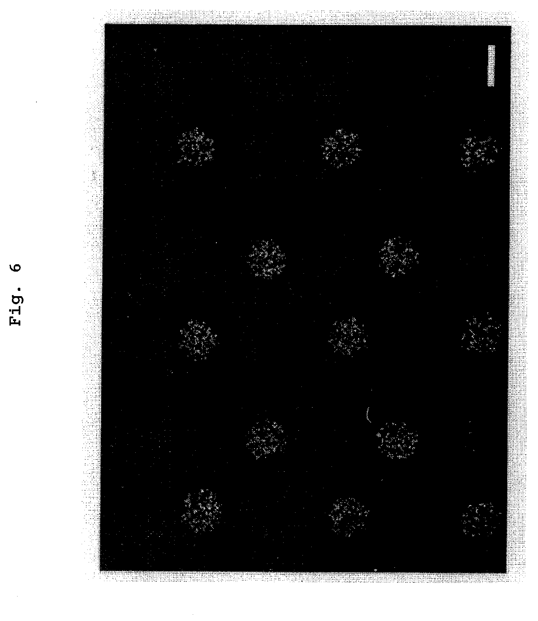

The GFP expression 48 hours after electroporation was observed with a fluorescence microscope. The results are shown in FIG. 6. As shown in Fig. 6, the GFP expression was site-specifically observed.

### Example 5

### 1. Preparation of CNT-loaded transfection array

According to 1 of Example 4, a CNT-loaded transfection array was prepared.

### 2. Introduction of site-specific small interfering RNA (siRNA) by CNT-loaded transfection array

Used were siRNAs which specifically suppress the expression of GFP sequence (GFP-siRNA sense: 5'-GCAAGCUGACCCUGA AGUUCAU-3' (SEQ ID NO: 1) and antisense: 5'-GAACUUCAGGGUCAGCUUGCCG-3' (SEQ ID NO: 2). Solutions (5 µg/mL and 50 µg/mL) of GFP-siRNA in PBS (pH 7.4) were spotted. HEK293 cells which stably express GFP was seeded onto the CNT-loaded transfection array at 6.0 × 10⁴ cells/cm², and for sufficient cell adhesion, incubation was carried out in 5% CO₂ at 37°C for 24 hours. Pulse voltage at 13V was applied once for 10 ms to the CNT-loaded transfection array onto which cells adhere for introduction of the siRNA. As a control, siRNAs which are non-specific to GFP sequence (control-siRNA, sense: 5'-UUCUCCGAACGUGUCACGUdTdT-3' (SEQ ID NO: 3) and antisense: 5'-ACGUGACACGUUCGGAGAA dTdT-3' (SEQ ID NO: 4) were used.

### 3. GFP expression suppression analysis

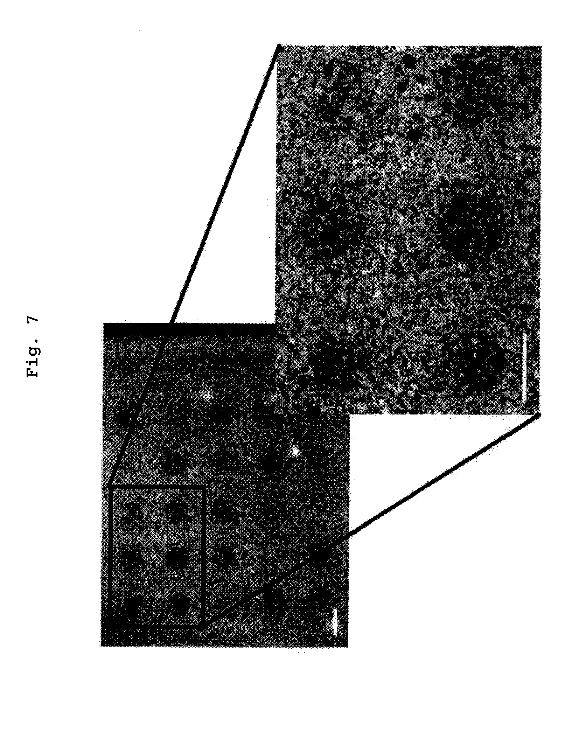

According to 4 of Example 1, suppression of GFP expression was observed. The results are shown in FIG. 7. As shown in Fig. 7, the GFP expression was site-specifically suppressed by GFP-siRNA.

### Example 6

It is expected that transfection efficiency and the amount of gene expression are dependent on the amount of CNT on an electrode surface. CNT-COOH and linear polyethyleneimine (L-PEI) were alternately laminated to prepare various electrode substrates having different numbers of times of CNT-COOH/L-PEI lamination, and the amount of gene expression was examined in each case.

### 1. Preparation of electrode with gold surface on which an anionic self-assembled monolayer (SAM) film is formed and further on which CNT-COOH and cationic polymer are alternately laminated

According to 2 of Example 1, a SAM film with a carboxyl group was formed on a gold thin film deposited substrate. Then, the substrate was immersed in a 1 mg/mL solution of linear polyethyleneimine having a molecular weight of 25,000 (L-PEI25000) in PBS (pH: 7.4) for 30 minutes, and a cationic substrate (L-PEI25000/COOH-SAM) was obtained. The substrate was immersed in a 0.1 mg/mL dispersed solution of CNT-COOH in purified water for 2 hours, and CNT-COOH was loaded on the substrate (CNT/L-PEI25000/COOH-SAM). This operation was performed several times to prepare an electrode on which CNT-COOH and L-PEI25000 were alternately laminated. The prepared electrode was expressed as (CNT/L-PEI25000)ₙ/COOH-SAM (n; number of times of alternate lamination) according to the number of times of alternate lamination.

### 2. Gene introduction and gene expression analysis

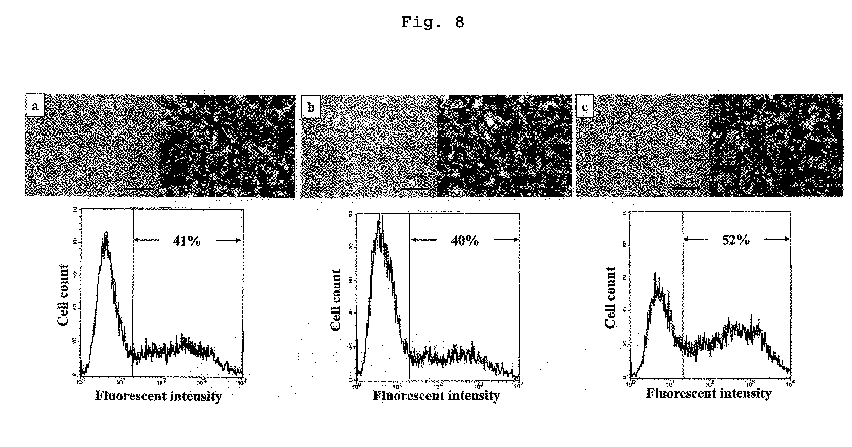

According to 3 of Example 1, plasmid adsorption and cell seeding were performed using the obtained electrodes. However, the concentration of the pEGFP-C1 solution and the contact time with the electrodes were 5 µg/mL and 3 hours, respectively. Cell seeding was performed at 2.0 × 10⁴ cells/cm². According to 3 of Example 1, electroporation was performed using the above-mentioned electrodes. According to 4 of Example 1, the GFP expression was analyzed with a fluorescence microscope and by flow cytometry. The results are shown in Fig. 8. In Fig. 8, (a) is a result of a CNT/L-PEI25000/COOH-SAM electrode, (b) is a result of a L-PEI25000/(CNT/L-PEI25000)₁/COOH-SAM electrode, and (c) is a result of a (CNT/L-PEI25000)₅/COOH-SAM electrode.

### 3. GFP expression analysis

As shown in Fig. 8, in the cases of cells adherent to electrodes with fewer times of lamination (Fig. 8 (a) and (b)), the gene expression efficiencies were both about 40%, and no significant difference was found between the electrodes. However, in the cells on the electrode with more times of lamination (Fig. 8 (c)), the gene expression efficiency was about 50%. By alternately laminating CNT-COOH and L-PEI25000 to increase the amount of loaded CNT, the transfection efficiency was improved.

### INDUSTRIAL APPLICABILITY

The conductive substrate and the method of the present invention for introducing a nucleic acid enable efficient transfection without damaging cells. Therefore, a significant functional analysis can be carried out using only a small number of cells, the timing and site of transfection can be restricted, and functional analysis of a wide variety of nucleic acids can be conducted simultaneously. Therefore, the conductive substrate and the method of the present invention for introducing a nucleic acid enables efficient functional analysis of genes and gene products at the cellular level, greatly promotes research on cellular biology, post-genome, and proteome, and makes great contribution to medical science.

## Claims

1. A conductive substrate for introducing a nucleic acid into a cell, which comprises a carbon nanotube with a carboxyl group, and a nucleic acid, the carbon nanotube and the nucleic acid being loaded on an electrode substrate with a cationic surface.

2. The conductive substrate for introducing a nucleic acid according to claim 1, wherein the carbon nanotube with a carboxyl group, and the nucleic acid are loaded on the cationic surface of the electrode substrate directly or via a cationic polymer.

3. The conductive substrate for introducing a nucleic acid according to claim 2, wherein the carbon nanotube with a carboxyl group is made to adsorb onto the cationic surface of the electrode substrate only once, or the carbon nanotube with a carboxyl group and the cationic polymer are alternately laminated on the cationic surface of the electrode substrate in the order of a carbon nanotube with a carboxyl group, a cationic polymer, and a carbon nanotube with a carboxyl group.

4. The conductive substrate for introducing a nucleic acid according to claim 2 or 3, wherein the nucleic acid is made to adsorb only once, or the nucleic acid and the cationic polymer are alternately laminated in the order of a nucleic acid, a cationic polymer, and a nucleic acid.

5. The conductive substrate for introducing a nucleic acid according to any one of claims 1 to 4, wherein the electrode substrate with a cationic surface has a substrate coated with a metal selected from gold, platinum and aluminum, or with a transparent semiconductor film of indium tin oxide (ITO).

6. The conductive substrate for introducing a nucleic acid according to any one of claims 1 to 5, wherein the electrode substrate with a cationic surface has a substrate coated with a gold thin film.

7. The conductive substrate for introducing a nucleic acid according to any one of claims 1 to 6, wherein the electrode substrate with a cationic surface has a monolayer film of a thiol compound, a disulfide compound, a sulfide compound or a silane coupling agent having a cationic functional group at the terminal, the monolayer film being formed on the surface of an electrode, or wherein the surface of the electrode substrate is modified with a cationic polymer.

8. The conductive substrate for introducing a nucleic acid according to any one of claims 1 to 7, wherein the electrode substrate with a cationic surface has a micropatterned surface.

9. The conductive substrate for introducing a nucleic acid according to any one of claims 1 to 8, wherein the nucleic acid is at least one kind selected from the group consisting of DNAs, RNAs, antisense nucleic acids, siRNAs, and expression vectors therefor.

10. A method for introducing a nucleic acid into a cell by electroporation, which comprises the steps of:
(A) loading a carbon nanotube with a carboxyl group, and a nucleic acid onto the surface of an electrode;
(B) making a cell adhere to the surface of the obtained nucleic acid-loaded electrode; and
(C) applying an electrical pulse to the adherent cell.
